# EUROPEAN PATENT SPECIFICATION

(11) **EP 3 659 443 B1**
(45) Date of publication and mention of the grant of the patent: **06.04.2022**
(21) Application number: 18838238.6
(22) Date of filing: 27.07.2018
(51) Int. Cl.: A23L 2/00, A23C 9/156, A23L 2/38, C11B 9/00, A23G 3/36, A23G 4/06, A23L 2/56, A23L 27/20

(54) **BEVERAGE AND FOOD**
GETRÄNK UND NAHRUNGSMITTEL
BOISSON ET ALIMENT

(30) Priority: 28.07.2017 JP 2017146168
(43) Date of publication of application: 03.06.2020
(73) Proprietor: Takasago International Corporation, Tokyo 144-8721 (JP)
(72) Inventor: YAMAZAKI, Yuichiro, Hiratsuka-shi Kanagawa 254-0073 (JP); CHIBA, Shingo, Hiratsuka-shi Kanagawa 254-0073 (JP); GONDA, Yoshiharu, Hiratsuka-shi Kanagawa 254-0073 (JP); UJIHARA, Hideo, Hiratsuka-shi Kanagawa 254-0073 (JP); SATO, Tomoharu, Hiratsuka-shi Kanagawa 254-0073 (JP); HABU, Ayano, Hiratsuka-shi Kanagawa 254-0073 (JP); MAEDA, Takehiro, Hiratsuka-shi Kanagawa 254-0073 (JP); HOSHINO, Kunihide, Hiratsuka-shi Kanagawa 254-0073 (JP)
(74) Representative: Zimmermann & Partner Patentanwälte mbB
(86) International application number: PCT/JP2018/028263
(87) International publication number: WO 2019/022233

(56) References cited:
- WO-A1-2018/206415
- JP-A- 2010 088 348
- JP-A- 2012 050 347
- JP-A- 2016 198 025
- US-A1- 2008 064 624
- Yamamoto, Takeshi et al.: "Olfactory study on optically active citronellyl derivatives", Flavourand Fragrance Journal, vol. 19, 8 March 2004 (2004-03-08), pages 121-133, XP009069049, DOI: 10.1002/ffj.1280
- "List of foodstufs (non-official translation)", Japan Flavor & Fragrance Materials Association , 26 February 2014 (2014-02-26), XP055665305, Retrieved from the Internet: URL:http://www.jffma-jp.org/flavor/safety/ f-list.html [retrieved on 2018-08-22]

## Description

### Technical Field

The present invention relates to a food and/or beverage and a food flavoring composition comprising 3,7-dimethyl-2-methylene-6-octen-1-ol and in particular to a food and/or beverage and a food flavoring composition to which an excellent citrus-like flavor is imparted.

### Background Art

3,7-Dimethyl-2-methylene-6-octen-1-ol used in the present invention is a known compound, and is described as having fragrance of fresh citrus peel and citrus odour with aliphatic aldehyde in Flavour Fragr. J., (2004), 19, 121-133. Meanwhile, GB 1136345 A states that 3,7-dimethyl-2-methylene-6-octen-1-ol has floral, rose-muguet odor. However, these literatures disclose that 3,7-dimethyl-2-methylene-6-octen-1-ol can be used as a fragrance for perfumes and/or cosmetics, but do not state anything about use of it as a food flavoring ingredient in foods and/or beverages.

### Summary of Invention

An object of the present invention is to provide a food and/or beverage and a food flavoring composition which comprises 3,7-dimethyl-2-methylene-6-octen-1-ol and to which an excellent flavor property, in particular, a citrus flavor is imparted.

As a result of study for achieving the above object, the present inventors have found that a food and/or beverage and a food flavoring composition having an excellent flavor can be obtained when 3,7-dimethyl-2-methylene-6-octen-1-ol expressed by the following formula (1) is added as a food flavoring ingredient, and therefore have completed the present invention.

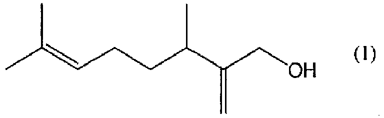

That is, the present invention comprises the following [1] to[7].
[1] A food or beverage comprising 3,7-dimethyl-2-methylene-6-octen-1-ol.
[2] The food or beverage according to the above [1], which is an acidic beverage.
[3] The food or beverage according to the above [1] or [2], wherein the 3,7-dimethyl-2-methylene-6-octen-1-ol is contained at 1 ppb to 1000 ppm (concentration).
[4] Use of 3,7-dimethyl-2-methylene-6-octen-1-ol as a flavoring substance in a food or beverage.
[5] The use according to the above [4], wherein the food or beverage is an acidic beverage.
[6] The use according to the above [4] or [5], wherein the 3,7-dimethyl-2-methylene-6-octen-1-ol is contained at 1 ppb to 1,000 ppm (concentration).
[7] A food flavoring composition comprising 3,7-dimethyl-2-methylene-6-octen-1-ol.

### Description of Embodiments

3,7-Dimethyl-2-methylene-6-octen-1-ol and the food flavoring composition of the present invention can be directly added to foods and beverages to impart or enhance a flavor, or also can be mixed with other ingredients. The other flavoring ingredients that can be mixed include various kinds of ingredients commonly used in this field, such as synthetic flavors, natural flavors, natural essential oils, and plant extracts, and are, for example, natural essential oils, natural flavors, synthetic flavors and so on described in "Official Gazette of Japan Patent Office, Collection of Well-known Prior Arts (Flavor and/or Fragrance), Chapter II Food Flavor, pp. 88-131, issued on January 14, 2000".

In addition, as 3,7-dimethyl-2-methylene-6-octen-1-ol, there are (3R) and (3S) optically active isomers and a racemate, any of which can be used in the present invention.

The content of 3,7-dimethyl-2-methylene-6-octen-1-ol in the food flavoring composition of the present invention varies depending on other flavoring ingredients mixed and cannot be mentioned unconditionally, but may be usually at a concentration range of 0.0001% to 50%, preferably 0.001% to 10%, and more preferably 0.01% to 5% with respect to the weight of the food flavoring composition. When the concentration of 3,7-dimethyl-2-methylene-6-octen-1-ol is less than 0.0001%, the effect of imparting or enhancing aroma and flavor of the present invention cannot be obtained. Meanwhile, when the concentration exceeds 50%, 3,7-dimethyl-2-methylene-6-octen-1-ol may impair the balance of the flavoring composition as a whole. Both cases are unfavorable.

Moreover, when 3,7-dimethyl-2-methylene-6-octen-1-ol is added to a food or beverage, the concentration range may be 1 ppb to 5000 ppm, preferably 10 ppb to 1000 ppm, and more preferably 100 ppb to 100 ppm with respect to the weight of the food or beverage. When the concentration of 3,7-dimethyl-2-methylene-6-octen-1-ol is less than 1 ppb, the effect of imparting or enhancing aroma and flavor of the present invention cannot be obtained. Meanwhile, when the concentration exceeds 5000 ppm, the floral scent inherent to 3,7-dimethyl-2-methylene-6-octen-1-ol may be felt too strong. Both cases are also unfavorable.

The food flavoring composition of the present invention comprising 3,7-dimethyl-2-methylene-6-octen-1-ol may contain generally-used ingredients as needed, for example, a solvent such as water or ethanol, and a fixative such as ethylene glycol, propylene glycol, dipropylene glycol, hexylglycol, benzyl benzoate, triethyl citrate, diethyl phthalate, hercoline, medium-chain fatty acid triglyceride, or medium-chain fatty acid diglyceride.

Specific examples of the foods and beverages to which the flavor can be imparted by addition of either of the food flavoring composition and 3,7-dimethyl-2-methylene-6-octen-1-ol of the present invention include: acidic beverages such as carbonated beverage, fruit juice beverage, sports drink, lactic acid bacteria beverage, fermented beverage, and alcoholic fruit drink; beverages such as dairy beverage, energy drink, soy milk, and tea beverage; desserts such as ice cream, ice milk, mellorine, sorbet, yogurt, pudding and jelly, and daily dessert; confectionery products such as caramel, candy, tablet, cracker, biscuit, cookie, pie, chocolate, snack food, chewing gum, steamed bean-jam bun, and Yokan; soups such as Japanese style soup, Western style soup, and Chinese style soup; bread; jam; flavor seasonings; various instant foods; and so on.

The amount of the food flavoring composition added to a food and/or beverage varies depending on the kind or form of a product, but may be usually at a concentration range of 0.001 to 10% and preferably 0.01 to 5% with respect to the weight of the product.

### Examples

Hereinafter, the present invention will be described in detail by using Examples, but the present invention should not be limited to these Examples.

### (Example 1)

Food flavoring compositions (lemon flavor) specified below in Table 1 were prepared. (S)-3,7-dimethyl-2-methylene-6-octen-1-ol used herein was produced in accordance with the method described in Flavour Fragr. J., (2004), 19, 121-133. All the numeric values in Table are of parts by weight.

**[Table 1]**

| Ingredient Name | Comparative Example 1 | Example 1 |
|---|---|---|
| (S)-3,7-dimethyl-2-methylene-6-octen-1-ol | - | 5.0 |
| Lemon Oil (Cold Pressed) | 50.0 | 50.0 |
| Ethanol | Balance | Balance |
| Total | 1000.0 | 1000.0 |

### (Text Example 1)

The food flavoring compositions obtained according to Table 1 were compared by five well-trained panelists. All the five panelists answered that Example 1 was remarkably superior because a natural fruit juice-like flavor which was not obtained by Comparative Example 1 was imparted by Example 1. The same result was also obtained when the (R) isomer and the racemate were used.

### (Test Example 2)

Each of the food flavoring compositions was added for flavoring at 0.1% to a commercially available lemon-flavored beverage and the flavors of the resultant beverages were evaluated by five well-trained panelists. All the five panelists answered that the flavor of the beverage supplemented with the flavoring composition of Example 1 was remarkably superior because a natural fruit juice-like and voluminous flavor was imparted. The same result was also obtained when the (R) isomer and the racemate were used.

### (Example 2)

(S)-3,7-dimethyl-2-methylene-6-octen-1-ol was added at 5 ppm to a commercially available lemon-flavored beverage and the flavor of the resultant beverage was evaluated by five well-trained panelists. All the five panelists answered that the flavor of the beverage supplemented with (S)-3,7-dimethyl-2-methylene-6-octen-1-ol was remarkably superior because a natural fruit juice-like and voluminous flavor was imparted. The same result was also obtained when the (R) isomer and the racemate were used.

### (Example 3)

Food flavoring compositions (grapefruit flavor) specified below in Table 2 were prepared. All the numeric values in Table are of parts by weight.

**[Table 2]**

| Ingredient Name | Comparative Example 2 | Example 3 |
|---|---|---|
| (S)-3,7-dimethyl-2-methylene-6-octen-1-ol | - | 2.0 |
| Grapefruit Oil (Cold Pressed) | 50.0 | 50.0 |
| Ethanol | Balance | Balance |
| Total | 1000.0 | 1000.0 |

### (Test Example 3)

The food flavoring compositions obtained according to Table 2 were compared by five well-trained panelists. All the five panelists answered that Example 3 was remarkably superior because a natural fruit juice-like flavor which was not obtained by Comparative Example 2 was imparted by Example 3. The same result was also obtained when the (R) isomer and the racemate were used.

### (Test Example 4)

Each of these food flavoring compositions was added for flavoring at 0.1% to a commercially available grapefruit-flavored beverage and the flavors of the resultant beverages were evaluated by five well-trained panelists. All the five panelists answered that the flavor of the beverage supplemented with the flavoring composition of Example 3 was remarkably superior because a natural fruit juice-like and voluminous flavor was imparted. The same result was also obtained when the (R) isomer and the racemate were used.

### (Example 4)

(S)-3,7-dimethyl-2-methylene-6-octen-1-ol was added at 2 ppm to a commercially available grapefruit-flavored sports drink and the flavor of the resultant drink was evaluated by five well-trained panelists. All the five panelists answered that the flavor of the drink supplemented with (S)-3,7-dimethyl-2-methylene-6-octen-1-ol was remarkably superior because a natural fruit juice-like and voluminous flavor was imparted. The same result was also obtained when the (R) isomer and the racemate were used.

### (Example 5)

Food flavoring compositions (orange flavor) specified below in Table 3 were prepared. All the numeric values in Table are of parts by weight.

**[Table 3]**

| Ingredient Name | Comparative Example 3 | Example 5 |
|---|---|---|
| (S)-3,7-dimethyl-2-methylene-6-octen-1-ol | - | 2.0 |
| Orange Oil (Cold Pressed) | 50.0 | 50.0 |
| Ethanol | Balance | Balance |
| Total | 1000.0 | 1000.0 |

### (Test Example 5)

The food flavoring compositions obtained according to Table 3 were compared by five well-trained panelists. All the five panelists answered that Example 5 was remarkably superior because a natural sweet fruit juice-like flavor which was not obtained by Comparative Example 3 was imparted by Example 5. The same result was also obtained when the (R) isomer and the racemate were used.

### (Test Example 6)

Each of these food flavoring compositions was added for flavoring at 0.1% to a commercially available orange-flavored beverage and the flavors of the resultant beverages were evaluated by five well-trained panelists. All the five panelists answered that the flavor of the beverage supplemented with the flavoring composition of Example 5 was remarkably superior because a natural sweet fruit juice-like and voluminous flavor was imparted. The same result was also obtained when the (R) isomer and the racemate were used.

### (Example 6)

(S)-3,7-dimethyl-2-methylene-6-octen-1-ol was added at 2 ppm to a commercially available orange-flavored beverage and the flavor of the resultant beverage was evaluated by five well-trained panelists. All the five panelists answered that the flavor of the beverage supplemented with (S)-3,7-dimethyl-2-methylene-6-octen-1-ol was remarkably superior because a natural sweet fruit juice-like and voluminous flavor was imparted. The same result was also obtained when the (R) isomer and the racemate were used.

### (Example 7)

Food flavoring compositions (banana flavor) specified below in Table 4 were prepared. All the numeric values in Table are of parts by weight.

**[Table 4]**

| Ingredient Name | Comparative Example 4 | Example 7 |
|---|---|---|
| (S)-3,7-dimethyl-2-methylene-6-octen-1-ol | - | 1.0 |
| Isoamyl Acetate | 50.0 | 50.0 |
| Isoamyl Butyrate | 20.0 | 20.0 |
| Cis-3-Hexenyl Acetate | 3.0 | 3.0 |
| Trans-2-Hexenal | 2.0 | 2.0 |
| Eugenol | 2.0 | 2.0 |
| Isoamyl Alcohol | 5.0 | 5.0 |
| Ethanol | Balance | Balance |
| Total | 1000.0 | 1000.0 |

### (Test Example 7)

The food flavoring compositions obtained according to Table 4 were compared by five well-trained panelists. All the five panelists answered that Example 7 was remarkably superior because a natural fresh fruit juice-like flavor which was not obtained by Comparative Example 4 was imparted by Example 7. The same result was also obtained when the (R) isomer and the racemate were used.

### (Test Example 8)

Each of these food flavoring compositions was added for flavoring at 0.1% to a commercially available banana-flavored dairy beverage and the flavors of the resultant beverages were evaluated by five well-trained panelists. All the five panelists answered that the flavor of the beverage supplemented with the flavoring composition of Example 7 was remarkably superior because a natural fresh fruit juice-like flavor was imparted. The same result was also obtained when the (R) isomer and the racemate were used.

### (Example 8)

(S)-3,7-dimethyl-2-methylene-6-octen-1-ol was added at 1 ppm to a commercially available banana-flavored dairy beverage and the flavor of the resultant beverage was evaluated by five well-trained panelists. All the five panelists answered that the flavor of the beverage supplemented with (S)-3,7-dimethyl-2-methylene-6-octen-1-ol was remarkably superior because a natural fresh fruit juice-like flavor was imparted. The same result was also obtained when the (R) isomer and the racemate were used.

### (Example 9)

Food flavoring compositions (lemon lime flavor) specified below in Table 5 were prepared. All the numeric values in Table are of parts by weight.

**[Table 5]**

| Ingredient Name | Comparative Example 5 | Example 9 |
|---|---|---|
| (S)-3,7-dimethyl-2-methylene-6-octen-1-ol | - | 2.0 |
| Isoamyl Acetate | 30.0 | 30.0 |
| Ethyl Butyrate | 10.0 | 10.0 |
| Vanillin | 0.5 | 0.5 |
| Lemon Terpeneless Oil | 5.0 | 5.0 |
| Lime Terpeneless Oil | 2.0 | 2.0 |
| Ethanol | Balance | Balance |
| Total | 1000.0 | 1000.0 |

### (Test Example 9)

The food flavoring compositions obtained according to Table 5 were compared by five well-trained panelists. All the five panelists answered that Example 9 was remarkably superior because fresh sweetness which was not obtained by Comparative Example 5 was imparted by Example 9. The same result was also obtained when the (R) isomer and the racemate were used.

### (Test Example 10)

Each of these food flavoring compositions was added for flavoring at 0.1% to commercially available carbonated water and the flavors of the resultant beverages were evaluated by five well-trained panelists. All the five panelists answered that the flavor of the beverage supplemented with the flavoring composition of Example 9 was remarkably superior because it had natural fresh sweetness. The same result was also obtained when the (R) isomer and the racemate were used.

### (Example 10)

(S)-3,7-dimethyl-2-methylene-6-octen-1-ol was added at 2 ppm to a commercially available lemon lime-flavored carbonated beverage and the flavor of the resultant beverage was evaluated by five well-trained panelists. All the five panelists answered that the flavor of the beverage supplemented with (S)-3,7-dimethyl-2-methylene-6-octen-1-ol was remarkably superior because natural fresh sweetness was imparted. The same result was also obtained when the (R) isomer and the racemate were used.

### (Example 11)

Food flavoring compositions (ginger ale flavor) specified below in Table 6 were prepared. All the numeric values in Table are of parts by weight.

**[Table 6]**

| Ingredient Name | Comparative Example 6 | Example 11 |
|---|---|---|
| (S)-3,7-dimethyl-2-methylene-6-octen-1-ol | - | 3.0 |
| Orange Terpeneless Oil | 1.0 | 1.0 |
| Lime Terpeneless Oil | 3.0 | 3.0 |
| Lemon Terpeneless Oil | 5.0 | 5.0 |
| Ginger Oil | 10.0 | 10.0 |
| Ethanol | Balance | Balance |
| Total | 1000.0 | 1000.0 |

### (Test Example 11)

The food flavoring compositions obtained according to Table 6 were compared by five well-trained panelists. All the five panelists answered that Example 11 was remarkably superior because a natural fresh ginger flavor which was not obtained by Comparative Example 6 was imparted by Example 11. The same result was also obtained when the (R) isomer and the racemate were used.

### (Test Example 12)

Each of these food flavoring compositions was added for flavoring at 0.1% to commercially available carbonated water and the flavors of the resultant beverages were evaluated by five well-trained panelists. All the five panelists answered that the flavor of the beverage supplemented with the flavoring composition of Example 11 was remarkably superior because it had ginger-like freshness. The same result was also obtained when the (R) isomer and the racemate were used.

### (Example 12)

(S)-3,7-dimethyl-2-methylene-6-octen-1-ol was added at 3 ppm to a commercially available carbonated beverage with a ginger ale flavor and the flavor of the resultant beverage was evaluated by five well-trained panelists. All the five panelists answered that the flavor of the beverage supplemented with (S)-3,7-dimethyl-2-methylene-6-octen-1-ol was remarkably superior because the fresh ginger flavor was enhanced. The same result was also obtained when the (R) isomer and the racemate were used.

### (Example 13)

Food flavoring compositions (lemon flavor) specified below in Table 7 were prepared. All the numeric values in Table are of parts by weight.

**[Table 7]**

| Ingredient Name | Comparative Example 7 | Example 13 |
|---|---|---|
| (S)-3,7-dimethyl-2-methylene-6-octen-1-ol | - | 20.0 |
| Citral | 50.0 | 50.0 |
| α-Terpineol | 5.0 | 5.0 |
| Geraniol | 5.0 | 5.0 |
| Geranyl Acetate | 5.0 | 5.0 |
| Neryl Acetate | 5.0 | 5.0 |
| Linalool | 3.0 | 3.0 |
| Octanal | 2.0 | 2.0 |
| Limonene | Balance | Balance |
| Total | 1000.0 | 1000.0 |

### (Test Example 13)

Each of these food flavoring compositions was added for flavoring at 1% to a chewing gum base specified below in Table 8 and the flavors of the resultant gums were evaluated by five well-trained panelists. All the five panelists answered that the flavor of the gum supplemented with the flavoring composition of Example 13 was remarkably superior because a natural fruit juice-like and voluminous flavor was imparted. The same result was also obtained when the (R) isomer and the racemate were used.

**[Table 8]**

| Ingredient Name | Content (g) |
|---|---|
| Xylitol | 320.0 |
| Maltitol | 348.8 |
| Gum Base | 280.0 |
| Reduced Starch Saccharified Product (BRIX70) | 40.0 |
| Glycerin | 10.0 |
| Acesulfame K | 0.6 |
| Aspartame | 0.6 |
| Total | 1000.0 |

### (Test Example 14)

Each of the food flavoring compositions in Table 7 was added for flavoring at 0.2% to a candy base specified below in Table 9 and the flavors of the resultant candies were evaluated by five well-trained panelists. All the five panelists answered that the flavor of the candy supplemented with the flavoring composition of Example 13 was remarkably superior because a natural fruit juice-like flavor with sweetness was imparted. The same result was also obtained when the (R) isomer and the racemate were used.

**[Table 9]**

| Ingredient Name | Content (g) |
|---|---|
| Granulated Sugar | 500.0 |
| Starch Syrup (BRIX85, 47DE) | 430.0 |
| Purified Water | 170.0 |
| Total | 1100.0 |

## Claims

1. A food or beverage comprising 3,7-dimethyl-2-methylene-6-octen-1-ol.

2. The food or beverage according to claim 1, which is an acidic beverage.

3. The food or beverage according to claim 1 or 2, wherein the 3,7-dimethyl-2-methylene-6-octen-1-ol is contained at 1 ppb to 5,000 ppm.

4. Use of 3,7-dimethyl-2-methylene-6-octen-1-ol as a flavoring substance in a food or beverage.

5. The use according to claim 4, wherein the food or beverage is an acidic beverage.

6. The use according to claim 4 or 5, wherein the 3,7-dimethyl-2-methylene-6-octen-1-ol is contained at 1 ppb to 5,000 ppm.

7. A food flavoring composition comprising 3,7-dimethyl-2-methylene-6-octen-1-ol.

## Patentansprüche

1. Nahrungsmittel oder Getränk, enthaltend 3,7-Dimethyl-2-methylen-6-octen-1-ol.

2. Nahrungsmittel oder Getränk nach Anspruch 1, welches ein säurehaltiges Getränk ist.

3. Nahrungsmittel oder Getränk nach Anspruch 1 oder 2, wobei das 3,7-Dimethyl-2-methylen-6-octen-1-ol zu 1 ppb bis 5.000 ppm enthalten ist.

4. Verwendung von 3,7-Dimethyl-2-methylen-6-octen-1-ol als Geschmacksstoff in einem Nahrungsmittel oder Getränk.

5. Verwendung nach Anspruch 4, wobei das Nahrungsmittel oder Getränk ein säurehaltiges Getränk ist.

6. Verwendung nach Anspruch 4 oder 5, wobei das 3,7-Dimethyl-2-methylen-6-octen-1-ol zu 1 ppb bis 5.000 ppm enthalten ist.

7. Lebensmittel aromatisierende Zusammensetzung, die 3,7-Dimethyl-2-methylen-6-octen-1-ol enthält.

## Revendications

1. Aliment ou boisson comprenant du 3,7-diméthyl-2-méthylène-6-octèn-1-ol.

2. L'aliment ou la boisson selon la revendication 1, qui est une boisson acide.

3. L'aliment ou la boisson selon la revendication 1 ou 2, sachant que le 3,7-diméthyl-2-méthylène-6-octèn-1-ol est contenu à raison de 1 ppb à 5 000 ppm.

4. Utilisation de 3,7-diméthyl-2-méthylène-6-octèn-1-ol comme substance aromatisante dans un aliment ou une boisson.

5. L'utilisation selon la revendication 4, sachant que l'aliment ou la boisson est une boisson acide.

6. L'utilisation selon la revendication 4 ou 5, sachant que le 3,7-diméthyl-2-méthylène-6-octèn-1-ol est contenu à raison de 1 ppb à 5 000 ppm.

7. Composition aromatisante d'aliment comprenant du 3,7-diméthyl-2-méthylène-6-octèn-1-ol.
